# EUROPEAN PATENT SPECIFICATION

(11) **EP 2 403 477 B1**
(45) Date of publication and mention of the grant of the patent: **15.01.2014**
(21) Application number: 10706647.4
(22) Date of filing: 04.03.2010
(51) Int. Cl.: A61K 9/107, A61K 47/44, A61K 47/18, A61K 31/5575, A61P 27/06, A61K 9/00

(54) **CATIONIC OIL-IN-WATER EMULSIONS CONTAINING PROSTAGLANDINS AND USES THEREOF**
KATIONISCHE ÖL-IN-WASSER-EMULSIONEN MIT PROSTAGLANDINEN UND VERWENDUNGEN DAFÜR
ÉMULSIONS CATIONIQUES D'HUILE DANS L'EAU CONTENANT DES PROSTAGLANDINES ET LEURS UTILISATIONS

(30) Priority: 04.03.2009 EP 09305202; 04.03.2009 US 157355 P
(43) Date of publication of application: 11.01.2012
(73) Proprietor: Novagali Pharma S.A., 91000 Evry (FR)
(72) Inventor: LALLEMAND, Frédéric, F-94260 Fresnes (FR); PHILLIPS, Betty, F-92160 Antony (FR); GARRIGUE, Jean-Sébastien, F-91370 Verrières le Buisson (FR)
(74) Representative: Icosa
(86) International application number: PCT/EP2010/052741
(87) International publication number: WO 2010/100218

(56) References cited:
- EP-A- 1 655 021
- EP-A- 1 994 933
- WO-A-2007/042262

## Description

The present invention pertains to cationic oil-in-water emulsions containing prostaglandins for the topical administration of prostaglandins and in particular for the treatment of ophthalmic conditions or diseases, preferably ophthalmic conditions affecting the interior of the eye, more specifically the anterior segment of the eye, including ocular hypertension and/or glaucoma, and also for promoting growth of eyelashes and/or for treating eyelash hypotrichosis. The cationic oil-in-water emulsion according to the invention further presents the advantage to enhance the chemical stability of prostaglandins.

In the present invention, the term "prostaglandin" is indifferently used for prostaglandins, their derivatives, precursors, prodrugs or analogues.

Glaucoma is a disease characterized by an increase in the intraocular pressure (IOP) and is often associated with optic nerve damage and visual field defect. If left untreated, glaucoma can ultimately lead to blindness.

Prostaglandins, especially prostaglandin F₂ₐₗₚₕₐ and its phenyl-substituted analogues, have been shown to effectively reduce the IOP in man and animals. In fact, they have been used in ophthalmic preparations in order to treat glaucoma. For instance, latanoprost is available in the form of a topical eye solution (eyedrops) and sold under the trademark Xalatan^{®}.

Indeed, latanoprost is a potent prostaglandin F₂ₐₗₚₕₐ analogue which has been developed for the treatment of glaucoma. Its chemical name is isopropyl - (Z)-7-[(1R,2R,3R,5S)-3,5-dihydroxy-2-[(3R)3-hydroxy-5-phenylpentyl]-cyclopentyl]-5-heptenoate, its molecular formula is C₂₆H₄₀O₅ and its chemical structure is:

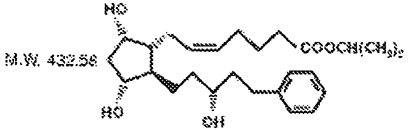

Specifically, latanoprost is a lipophilic prodrug in which the carboxylic acid moiety in the α-chain has been esterified to increase the bioavailability of the active drug into the eye. In addition, latanoprost is absorbed through the cornea where the isopropyl ester prodrug is hydrolyzed to the acid form to become biologically active.

Some ophthalmic prostaglandins, such as bimatoprost, latanoprost or travoprost, have also been described as being capable of promoting eyelash growth. Such prostaglandins could therefore be used for the topical treatment of eyelash hypotrichosis.

The problem generally encountered with prostaglandins is that they may be chemically unstable. In particular, latanoprost is known to be very sensitive towards light and heat. Indeed, these two elements (i.e. light and heat) may have an impact on the stability of latanoprost by provoking its hydrolyzation and/or oxidation. Consequently, unopened bottles of Xalatan^{®} should be stored in the dark and under refrigeration at 2-8 °C.

Consequently, there is a need for prostaglandin formulations which show an enhanced chemical stability of the prostaglandin and, in particular, an enhanced stability overtime towards light and heat.

The Applicant already conceived prostaglandin emulsions, and found that emulsions were a suitable vehicle to stabilize prostaglandins (see for example WO2007/042262).

However, there is a constant research for improvement in the field of ophthalmic emulsions, and the Applicant surprisingly experimented that stability of prostaglandins could be achieved with very low amounts of surfactants thus avoiding drawbacks due to high amounts of surfactants, often responsible of eye irritations or itching.

The present invention provides a prostaglandin composition which exhibits an improved stability of the prostaglandin compared to commercial products, while at the same time being light in surfactants, non toxic, tolerable for the patient and at least as efficient as the commercially available products.

An object of the present invention is a colloidal cationic oil-in-water emulsion comprising:
- a prostaglandin,
- an oil having a iodine value ≤ 2,
- one or more surfactants including one quaternary ammonium compound, which is cetalkonium chloride,
- water,
wherein the prostaglandin/total sum of surfactants mass ratio is comprised between 0.5 and 5.

In particular embodiments of the present invention, the prostaglandin/total sum of surfactants mass ratio in the emulsion ranges from 0.5 to 4, or from 0.5 to 3, or ranges from 0.5 to 3, or ranges from 0.5 to 2, or ranges from 0.6 to 1.5, or ranges from 0.7 to 1.3, or is around 1. In the meaning of this invention, the term "around" preceding a selected value means ranging from plus or minus 10% of the selected value.

According to an embodiment, the emulsion includes prostaglandin, oil and surfactant(s), and is such that the ratio of the oil mass to the total mass of surfactants ranges from 50 to 500, preferably 100 to 400, more preferably around 200.

According to a preferred embodiment, in the emulsion, the prostaglandin/total sum of surfactants mass ratio ranges from 0.5 to 4, and the oil/total sum of surfactants mass ratio ranges from 50 to 500.

Surprisingly, the emulsions of the present invention show a remarkable stability although they contain very low amounts of surfactants and consequently an enhanced tolerability for patients.

According to the invention, "good tolerability" or "enhanced tolerability" means that the ratio "therapeutic benefit" to "ocular discomfort" is acceptable by the patient, and preferably similar to a placebo or NaCl solution 0.9%. It is generally accepted that in order to show good ocular tolerability the cationic agent content within the formulation should not exceed 0.1%, preferably not exceed 0.05% and even more preferably should not exceed 0.03%.

According to a preferred embodiment, the emulsion according to the present invention comprises one or more surfactants, including at least one quaternary ammonium compound

In the emulsion according to the present invention, the quaternary ammonium compound is used as a cationic agent.

The quaternary ammonium compound is cetalkonium chloride.

According to the invention, the emulsion includes only one surfactant, which is a quaternary ammonium compound, being cetalkonium chloride. In this embodiment, the emulsion of the invention is a more simple emulsion than in prior art, i.e. containing less ingredients than in prior art emulsions.

The concentration of the quaternary ammonium compound, preferably of cetalkonium chloride, is preferably comprised between 0.001 and 0.1 % w/w, more preferably between 0.002 and 0.05% w/w and even more preferably between 0.002 and 0.03% w/w.

Typically, in addition to quaternary ammonium surfactants, the emulsion may include further surfactants, such as for example further cationic surfactants, anionic surfactants, nonionic surfactants, zwitterionic surfactants, hydrophilic surfactants (with a high HLB), hydrophobic surfactants (with a low HLB) or mixtures thereof, as long as the zeta potential of the invention remains positive.

The further cationic surfactants comprise C10-C24 primary alkylamines, such as oleylamine or stearylamine, tertiary aliphatic amines, cationic lipids, amino alcohols, biguanide salts chosen from chlorhexidine and salts thereof, polyaminopropyl biguanide, phenformin, alkylbiguanide or mixtures thereof, cationic polymers selected from chitosan, 1,2-dioleyl-3-trimethylammonium-propane, 1, 2-dioleoyl-sn-glycero-phosphatidylethanolamine, cationic glycosphingo-lipids or cationic cholesterol derivatives, or mixtures thereof.

Typically, the nonionic surfactants comprise alkyl polyethylene oxide, alkylphenol polyethylene oxide, poloxamers, tyloxapol, alkyl polyglucosides, fatty alcohols, cocamide MEA, cocamide DEA, polyoxyethylene castor oil derivatives, sorbitan esters, polyoxyl stearates, polysorbates or mixtures thereof. According to another embodiment, the composition of the invention is free of polyoxyethylene castor oil derivatives.

Typically, the anionic surfactants comprise perfluorooctanoate, perfluorooctanesulfonate, alkyl sulphate salts, sodium lauryl ether sulphate, alkyl benzene sulfonate, soaps or fatty acid salts or mixtures thereof.

Typically, the zwitterionic surfactants comprise dodecyl betaine, cocamidopropyl betaine, coco-ampho-glycinate or mixtures thereof.

Typically, the surfactant according to the invention comprises hydrophilic surfactants (with a high HLB) and /or hydrophobic surfactant (with a low HLB) or mixtures thereof.

Preferably, the emulsion is free of phospholipids.

In a particular embodiment, the surfactants present in the emulsion of the invention are chosen among, poloxamers, tyloxapol, polysorbates, sorbitan esters, polyoxyl stearates or mixtures thereof.

In a particular embodiment, the emulsion comprises latanoprost, medium chain triglycerides (MCT), cetalkonium chloride as the only surfactant.

The emulsion according to the invention is a cationic emulsion, which means that the zeta potential remains positive overtime. This positive zeta potential may preferably be higher than 10 mV, more preferably be higher or equal to 20 mV.

It has long been recognised that the zeta potential is a very good index of the magnitude of the interaction between colloidal particles and measurements of zeta potential are commonly used to assess the stability of colloidal systems. The zeta potential measured in a particular system is dependent on the chemistry of the surface, and also of the way it interacts with its surrounding environment.

Typically, the cationic emulsions according to the invention are physically stable overtime and may keep a positive zeta potential over a period of two years at 25°C. For each measurement of the zeta potential, it is operated as follows:
The zeta potential of the emulsion droplet surface is determined by electrophoretic mobility in an apparatus such as a Malvern Zetasizer 2000 (Malvern Instruments, UK) equipped with suitable software and calibrated with the supplied standard.

The emulsion is diluted in double distilled water if needed in order to obtain the scattering intensity allowing optimal particle detection. The sample count rate should be between 100 to 1000 KCps, in homodyne detection (if heterodyne detection is used, the contribution of the reference beam should be deduced). Three consecutive measurements are performed at 25°C using a constant cell drive of 150mV. The electrophoretic mobility is converted into zeta potential values through the Smoluchowsky equation, using the dielectric constants and viscosity of water. The measured value corresponds to the average of the 3 obtained values.

In a particular embodiment, the emulsion of the invention is free of any buffer.

According to a particular embodiment of the present invention, the emulsion remains stable during autoclaving. According to the present invention, "autoclaving" is defined as sterilization of a product by steam under pressure, by heating said product in an autoclave at high temperatures (e.g. 100 to 200°C, preferably 121°C) during an extended period of time (e.g. 10 to 60 minutes, preferably 10 to 20 minutes) at around 103 kPa (15 psi) above atmospheric pressure. The steam and pressure transfer sufficient heat into organisms to kill them and thus sterilize the product.

According to the invention, "stability" is defmed as the extent to which a product retains, within specified limits and throughout its period of storage and use (i.e., its shelf life), the same properties and characteristics that it possessed at the time of manufacture.

The purpose of stability testing is to provide evidence concerning the quality of a drug substance or a drug product overtime, said product being subjected to a variety of environmental factors such as temperature, humidity and light. The result may be helpful in providing appropriate storage conditions, re-testing periods and shelf lives.

Although conventional stability studies do evaluate those factors which ultimately affect the expiration date of the drugs, these conventional studies are time and cost-consuming. Consequently, in order to predict shelf-life of a pharmaceutical product for example, the pharmaceutical industry usually uses "accelerated stability studies" (Stress Test). These accelerated studies help understand the intrinsic stability mechanism of the molecule of interest by establishing degradation pathways and by identifying the likely degradation products. In these types of studies, the products are usually subjected to extreme conditions, such as temperature of about 40°C for approximately 6 months.

According to the invention, "colloidal" means that the emulsion comprises colloid particles having an oily core surrounded by an interfacial film dispersed in water with a particle size equal or less than 1 µm. Typically, the oily core comprises a prostaglandin and an oil. The prostaglandin being lipophilic, it is thus understandable that it is essentially present in the oily core. Typically, the emulsion may contain other ingredients, such as emollients, preferably glycerol, pH adjusters, such as NaOH, osmotic agents or preservatives.

The cationic emulsion according to the invention such that the colloidal particles have an average particle size of equal or less than 1 µm, advantageously equal or less than 300 nm, more advantageously in the range of 100 to 250 nm.

In one embodiment, the prostaglandin is a prostaglandin F₂ₐₗₚₕₐ, a derivative, precursor, prodrug or analogue thereof.

In another embodiment, the prostaglandin is an ophthalmic prostaglandin, in particular a prostaglandin which is effective in treating ocular hypertension and/or glaucoma.

In another embodiment, the prostaglandin is an ester prodrug, an amide prodrug of an active prostaglandin, in particular of an active ophthalmic prostaglandin or a mixture thereof. Ester prodrugs include C₁-C₄ alkyl ester prodrugs, such as methyl ester, ethyl ester, isopropyl ester or butyl ester and amide prodrugs include C₁-C₄ alkyl amide prodrugs, such as methyl amide, ethyl amide, isopropyl amide or butyl amide.

According to a particular embodiment, the prostaglandin of the present invention is chosen among latanoprost, isopropyl unoprostone, travoprost, bimatoprost, tafluprost, or mixtures thereof.

In a preferred embodiment, the emulsion according to the present invention comprises latanoprost.

The amount of prostaglandin present in the oily core of the emulsion according to the invention depends on the nature of the prostaglandin and to the intended use. Typically, the amount of prostaglandin relative to the total weight of the emulsion is comprised between 0.001 to 1% w/w, preferably ranging from 0.002 to 0.3% w/w and even more preferably from 0.004 to 0.15% w/w.

In a particular embodiment, the prostaglandin can be combined with other antiglaucoma active ingredients, such as dorzolamide or timolol.

According to the present invention, the oil is preferably chosen among saturated oils which are able to limit the degradation of the prostaglandin by oxidation and/or hydrolysis in the emulsion.

According to the invention "saturated oil" is an oil which has an iodine value of less or equal to 2, preferably less than 2, which means that the oil is substantially free of any molecule having a hydrocarbon chain containing double or triple bonds.

The iodine value can be measured for example according to the methods disclosed in the European Pharmacopeia monograph 2.5.4 or US Pharmacopeia monograph 401.

According to a particular embodiment of the present invention, the oil is chosen among oily fatty acids, oily fatty alcohols, fatty acids esters, such as isopropyl myristate and isopropyl palmitate, vegetable oils, animal oils, mineral oils such as petrolatum, liquid paraffin, semi-synthetic oils such as fractionated oils obtained from vegetable oils or mixtures thereof.

According to the invention "semi-synthetic oils" are prepared by chemical synthesis from natural oils.

Particularly, the oil according to the invention is a semi-synthetic oil obtained from fractionated coconut oil, kernel oil or babassu oil. More particularly, the oil is medium chain triglycerides (MCT).

Indeed, according to the European Pharmacopeia, medium chain triglycerides (MCT) is described as the fixed oil extracted from the hard, dried fraction of the endosperm of *Cocos nucifera* L. by hydrolysis, fractionation of the fatty acids obtained, and re-esterification. MCT consists of a mixture of exclusively short- or medium-chain triglycerides of fatty acids, of which not less than 95% are the saturated fatty acids octanoic (caprylic) acid and decanoic (capric) acid.

Moreover, MCT can also be found in substantial amounts in kernel oil and babassu oil, in addition to some animal products, such as milk-fat, which may contain small amounts (up to 4%) of MCT.

In some embodiments, the amount of the oil relative to the total weight of the emulsion is not higher than 7% w/w, preferably between 0.5 and 5% w/w and even more preferably between 1 and 3%w/w.

In another embodiment, the pH of the emulsion is preferably comprised between 4 and 7, preferably ranging from 4.5 and 6.5 and more preferably ranging from 4.5 and 6, even more preferably around 5.

According to a preferred embodiment, the composition of the invention includes an osmotic agent, preferably glycerol.

Another object of the present invention is a process for manufacturing the emulsion previously described. The emulsions of the invention are therefore prepared according to the following steps:
- preparation of the oily phase by mixing the prostaglandin (e.g. latanoprost) with the saturated oil (e.g. MCT) and possibly the quaternary ammonium when it is not soluble in the aqueous phase,
- preparation of the aqueous phase by mixing the water-soluble ingredients (e.g. glycerol,) with purified water;
- incorporating the oily phase to the aqueous phase;
- decreasing the emulsion droplet size by any suitable means known to the man skilled in the art, for example by high-shear mixing;
- homogenizing the cooled emulsion;
- optionally, adjusting the pH to a physiological pH, by using for example NaOH or HCl;
- optionally sterilizing the emulsion by autoclaving.

An additional advantage of the emulsions of the invention is that they are stable during the autoclaving, in other words, the prostaglandin is not degraded and the zeta potential remains positive.

According to the present invention, the cationic oil-in-water emulsion according to the invention is useful for the treatment of patients suffering from an ophthalmic condition or disease, preferably an ophthalmic condition or disease of the interior of the eye. In an embodiment of the invention, said patients are not suffering from lesions of the ocular surface, especially of the cornea or the conjunctiva. In the meaning of this invention, a corneal or conjunctival lesion is a local destruction of corneal, conjunctival or goblet cells. Such lesions may be local or disseminated and result in corneal erosion, punctuate keratopathy, epithelial defects, corneal ulceration, corneal scarring, corneal thinning, corneal perforation, keratitis, conjunctivitis, wounds, tiny abrasions, etc.

In another embodiment, the patient is not intolerant to quaternary ammonium halides.

The emulsion according to the present invention is preferably intended to be applied topically, e.g. to the surface of the eye, especially on cornea or conjunctiva, or to hairs, such as eyelashes of a patient.

Thus, the present invention is also directed to a method for treating a patient suffering from an ophthalmic condition or disease, preferably an ophthalmic condition or disease of the interior of the eye, by administering a therapeutic amount of the composition of the invention. The dose regimen being two drops every day or one drop every day on the eye surface. This treatment is a lifelong treatment.

According to the present invention, the cationic oil-in-water emulsion is useful for promoting growth of eyelashes or treating eyelash hypotrichosis.

According to an embodiment of the invention, the emulsion may be in the form of eye drops, eye ointment, or ophthalmic gel.

An object of the present invention is a delivery device comprising the cationic oil-in-water emulsion according to the invention.

Typically the delivery device according to the invention is selected from the group comprising lenses, ocular patch, implant, insert.

Other features and advantages of the invention will emerge upon reading the following non limiting examples.

### BRIEF DESCRIPTION OF THE FIGURES

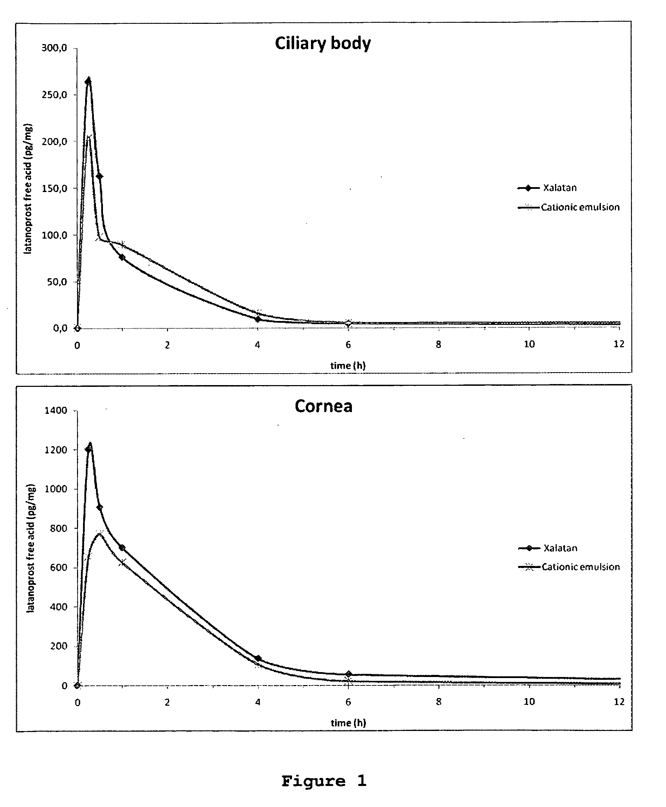

**Figure 1****:** Latanoprost free acid concentration in ciliary body and in cornea after administration of the emulsion of the invention.

### EXAMPLES

### 1. Preparation of a cationic oil-in-water emulsion.

The cationic oil-in-water emulsion according to the present invention is prepared by the following steps:
- preparation of the oily phase by mixing at 50°C the prostaglandin (latanoprost) with the saturated oil (MCT) and cetalkonium chloride respecting mass the ratio prostaglandin/total sum of surfactants of 1 and the ratio oil mass to the total mass of surfactants of 200.
- preparation of the aqueous phase by mixing at 50°C glycerol and purified water;
- incorporating the oily phase to the aqueous phase;
- decreasing the emulsion droplet size by any suitable means known to the man skilled in the art, for example by high-shear mixing 5 min at 16 000 rpm (Polytron PT6100; Kinematica, Switzerland)
- homogenizing during 20 min at 15 000 psi the cooled emulsion (Emulsiflex C3, Avestin, Canada)
- adjusting the pH with NaOH
- sterilizing the emulsion by autoclaving.

A composition of the emulsion is given in table 1.

**Table 1**

| | Ingredients | Supplier | Theorethical composition (% w/w) |
|---|---|---|---|
| Oily phase | MCT (Medium Chain Triglycerides) | Sasol GmbH, Germany | 1.000 |
| | Latanoprost | | 0.005 |
| | cetalkonium chloride | Dishman, India | 0.005 |
| Aqueous phase | Glycerol | Merck, Germany | 2.400 |
| | Water (up to 100) | | 96.590 |
| | NaOH 1M | Merck, Germany | qs pH 7 |
| | Total | | 100 % |

After sterilisation, the pH of the emulsion is around 5.0.

**Table 2**

| | Ingredients | Supplier | Theorethical composition (% w/w) |
|---|---|---|---|
| Oily phase | MCT (Medium Chain Triglycerides) | Sasol GmbH, Germany | 1.000 |
| | Travoprost | | 0.004 |
| | Cetalkonium chloride | Dishman, India | 0.005 |
| Aqueous phase | Glycerol | Merck, Germany | 2.400 |
| | Water (up to 100) | | 96.590 |
| | NaOH 1M | Merck, Germany | qs pH 7 |
| | Total | | 100 % |

### 2. Stability Test & Comparative Test

The stability of the emulsion of example 1 was evaluated under accelerated conditions "Stress Test" (at 80°C during 14 days), while a comparative analysis was conducted between the cationic emulsion (invention) and Xalatan^{®} under the same "Stress Test" conditions. Prostaglandin content was analysed in both tests by an HPLC-UV method. The results are given in table 2 (stability test) and table 3 (comparative test).

**Table 3**

| Emulsion of example 1 | Aspect | Zeta potential (mV) | Osmolality (mOsm/kg) | pH | Droplet size (nm) | Latanoprost (% w/w) |
|---|---|---|---|---|---|---|
| T= 0 days | White milky homogeneous emulsion Tyndall effect | 55.2 | 280 | 4.9 | 188 | 0.00511 |
| T= 7 days | White milky homogeneous emulsion Tyndall effect | 44.5 | 277 | 4.86 | 208 | - |
| T= 14 days | White milky homogeneous emulsion Tyndall effect | 42.5 | 281 | 4.88 | 221 | 0.00510 |

**Table 4**

| | Latanoprost (% w/w) | | pH | | Zeta potential (mV) | |
|---|---|---|---|---|---|---|
| | T0 | T 14 days | T0 | T14 days | T0 | T14 days |
| Emulsion of example 1 | 0.00511 | 0.00492 | 4.9 | 4.88 | 55.2 | 42.5 |
| Xalatan® | 0.00510 | 0.00248 | 6.74 | 6.71 | NA | NA |

| | | | | | | |
|---|---|---|---|---|---|---|
| NA : not applicable | | | | | | |

At T0, the concentrations in prostaglandins for the emulsion (invention) and for Xalatan^{®} are close to 0.005%. However, after subjecting both emulsions to the "Stress Test" (14 days at 80°C), it can be observed that the concentration of prostaglandins remains the same for the emulsion (invention), while it has decreased by more than half in the case of Xalatan^{®}. Furthermore, surprisingly, with a ratio of the oil mass to the total mass of surfactants of 200 (there is 200 times more oil than surfactant), the emulsion did not cream, did not coalesce nor separate.

### 3. Pharmacokinetic/pharmacodynamic studies of the emulsion of Table 1

Male and female New Zealand White rabbits were administrated with the emulsion of Table 1 or Xalatan^{®} and latanoprost free acid concentration was determined at different time points after administration (0.25, 0.5, 1, 4, 6 and 24 hour(s)) at the following target tissues: conjunctiva, cornea, aqueous humor and ciliary body. Tmax and AUC 0.5-24h were calculated and are presented hereafter in Table 5.

**Table 5**

| | Numerical field name | Units | Reference XALATAN^{®} | Cationic emulsion |
|---|---|---|---|---|
| Aqueous humor | Tₘₐₓ | h | 0.5-1 | 0.5-1 |
| | AUC_{0.25-24h} | pg x h/µl | 327 | 344 |
| Cornea | Tₘₐₓ | h | 0-0.25 | 0.5-1 |
| | AUC_{0.25-24h} | pg x h/mg | 2623 | 1925 |
| Conjunctiva | Tₘₐₓ | h | 0.25-0.5 | 0.5-1 |
| | AUC_{0.25-24h} | pg x h/mg | 197 | 174 |
| Ciliary body | Tₘₐₓ | h | 0-0.25 | 0.25-0.5 |
| | AUC_{0.25-24h} | pg x h/mg | 295 | 314 |

Figure 1 (ciliary body and cornea) and results here above presented show that latanoprost free acid is present at a high concentration in the target ocular tissues after administration of the emulsion. Said concentrations are similar to Xalatan(r) concentrations and are known to be sufficient to allow the opening of the Schlemm's canal and thus the evacuation of aqueous humor, thereby reducing the intraocular pressure.

## Claims

1. A colloidal oil-in-water emulsion comprising:
- a prostaglandin,
- an oil having a iodine value ≤ 2,
- one or more surfactants including one quaternary ammonium compound which is cetalkonium chloride,
- water,
wherein the emulsion has a positive zeta potential, and the prostaglandin/total sum of surfactants mass ratio is comprised between 0.5 and 5.

2. The colloidal cationic oil-in-water emulsion according to claim 1, **characterized in that** the prostaglandin/total sum of surfactants mass ratio is comprised between 0.5 and 4, preferably between 0.5 and 3, more preferably between 0.5 and 2, even more preferably between 0.6 and 1.5, even more preferably between 0.7 and 1.3, even more preferably around 1.

3. The colloidal cationic oil-in-water emulsion according to claim **1** or **2, characterized in that** the prostaglandin is chosen among an ester prodrug, an amide prodrug of an active prostaglandin or mixtures thereof.

4. The colloidal cationic oil-in-water emulsion according to anyone of claims **1** to **3, characterized in that** the prostaglandin is chosen among latanoprost, isopropyl unoprostone, travoprost, bimatoprost, tafluprost, or mixtures thereof.

5. The colloidal cationic oil-in-water emulsion according to anyone of claims **1** to **4, characterized in that** the oil is chosen among oily fatty acids, oily fatty alcohols, fatty acids esters, such as isopropyl myristate and isopropyl palmitate, vegetable oils, animal oils, mineral oils such as petrolatum, liquid paraffin, semi-synthetic oils such as fractionated oils obtained from vegetable oils or mixtures thereof.

6. The colloidal cationic oil-in-water emulsion according to claim 5, **characterized in that** the fractionated oil is medium chain triglycerides (MCT).

7. The colloidal cationic oil-in-water emulsion according to anyone of claims **1** to **6,** wherein the emulsion further includes surfactants selected from poloxamers, tyloxapol, polysorbates, sorbitan esters, polyoxyl stearates or mixtures thereof.

8. The colloidal cationic oil-in-water emulsion according to anyone of claims **1** to **7,** wherein the amount of prostaglandin relative to the total weight of the emulsion is comprised between 0.001 to 1% w/w, preferably between 0.002 to 0.3% w/w and even more preferably between 0.004 to 0.15% w/w.

9. The colloidal cationic oil-in-water emulsion according to anyone of claims **1** to **8,** wherein the amount of cetalkonium chloride relative to the total weight of the emulsion is comprised between 0.001 and 0.1% w/w, preferably between 0.002 and 0.05% w/w and even more preferably between 0.002 and 0.03% w/w.

10. The colloidal cationic oil-in-water emulsion according to anyone of claims **1** to **9,** wherein the amount of the oil relative to the total weight of the emulsion is not higher than 7% w/w, preferably between 0.5 and 5% w/w and even more preferably between 1 and 3% w/w.

11. The colloidal cationic oil-in-water emulsion according to anyone of claims **1** to **10,** wherein the prostaglandin/total sum of surfactants mass ratio ranges from 0.5 to 4, and the oil/total sum of surfactants mass ratio ranges from 50 to 500.

12. The colloidal cationic oil-in-water emulsion according to anyone of claims **1** to **11,** for use in the treatment of ocular hypertension and/or for the treatment of glaucoma.

13. Delivery device comprising the colloidal cationic oil-in-water emulsion according to anyone of claims **1** to **12.**

## Patentansprüche

1. Kolloidale Öl-in-Wasser-Emulsion, umfassend:
- ein Prostaglandin,
- ein Öl mit einer Jodzahl ≤ 2,
- ein oder mehrere oberflächenaktive Stoffe, umfassend eine quartemäre Ammoniumverbindung, wobei es sich um Cetalkoniumchlorid handelt,
- Wasser,
wobei die Emulsion ein positives Zeta-Potenzial aufweist und das Massenverhältnis zwischen Prostaglandin und der Gesamtsumme der oberflächenaktiven Stoffe zwischen 0,5 und 5 liegt.

2. Kolloidale kationische Öl-in-Wasser-Emulsion nach Anspruch **1, dadurch gekennzeichnet, dass** das Massenverhältnis zwischen Prostaglandin und der Gesamtsumme der oberflächenaktiven Stoffe zwischen 0,5 und 4 liegt, vorzugsweise zwischen 0,5 und 3, bevorzugter zwischen 0,5 und 2, noch bevorzugter zwischen 0,6 und 1,5, noch bevorzugter zwischen 0,7 und 1,3, noch bevorzugter ungefähr bei 1.

3. Kolloidale kationische Öl-in-Wasser-Emulsion nach Anspruch **1** oder **2, dadurch gekennzeichnet, dass** das Prostaglandin ausgewählt ist aus einem Ester-Prodrug, einem Amid-Prodrug eines aktiven Prostaglandins, oder Mischungen daraus.

4. Kolloidale kationische Öl-in-Wasser-Emulsion nach einem der Ansprüche **1** bis **3, dadurch gekennzeichnet, dass** das Prostaglandin ausgewählt ist aus Latanoprost, Isopropyl Unoprostone, Travoprost, Bimatoprost, Tafluprost oder Mischungen daraus.

5. Kolloidale kationische Öl-in-Wasser-Emulsion nach einem der Ansprüche **1** bis **4, dadurch gekennzeichnet, dass** das Öl ausgewählt ist aus ölhaltigen Fettsäuren, ölhaltigen Fettalkoholen, Fettsäureestern wie z.B. Isopropyl-Myristat und IsopropylPalmitat, pflanzlichen Ölen, tierischen Ölen, mineralischen Ölen wie z.B. Petrolatum, flüssigem Paraffin, halbsynthetischen Ölen wie z.B. fraktionierten Ölen erhalten aus pflanzlichen Ölen oder Mischungen daraus.

6. Kolloidale kationische Öl-in-Wasser-Emulsion nach Anspruch **5, dadurch gekennzeichnet, dass** es sich beim fraktionierten Öl um mittelkettige Triglyceride (MTC) handelt.

7. Kolloidale kationische Öl-in-Wasser-Emulsion nach einem der Ansprüche **1** bis **6,** wobei die Emulsion weiter oberflächenaktive Stoffe umfasst, ausgewählt aus Poloxameren, Tyloxapol, Polysorbaten, Sorbitanestern, Polyolstearaten oder Mischungen daraus.

8. Kolloidale kationische Öl-in-Wasser-Emulsion nach einem der Ansprüche **1** bis **7,** wobei die Menge von Prostaglandin mit Bezug auf das Gesamtgewicht der Emulsion zwischen 0,001 und 1 Gew% liegt, vorzugsweise zwischen 0,002 und 0,3 Gew%, und noch bevorzugter zwischen 0,004 und 0,15 Gew%.

9. Kolloidale kationische Öl-in-Wasser-Emulsion nach einem der Ansprüche **1** bis **8,** wobei die Menge von Cetalkoniumchlorid mit Bezug auf das Gesamtgewicht der Emulsion die zwischen 0,001 and 0,1 Gew% liegt, vorzugsweise zwischen 0,002 und 0,05 Gew% und noch bevorzugter zwischen 0,002 und 0,03 Gew%.

10. Kolloidale kationische Öl-in-Wasser-Emulsion nach einem der Ansprüche **1** bis **9,** wobei die Menge von Öl mit Bezug auf das Gesamtgewicht der Emulsion nicht höher als 7 Gew% ist, vorzugsweise zwischen 0,5 und 5 Gew% und noch bevorzugter zwischen 1 and 3 Gew%.

11. Kolloidale kationische Öl-in-Wasser-Emulsion nach einem der Ansprüche **1** bis **10,** wobei das Massenverhältnis zwischen Prostaglandin und der Gesamtsumme der oberflächenaktiven Stoffen von 0,5 to 4 reicht, und das Massenverhältnis zwischen Öl und der Gesamtsumme der oberflächenaktiven Stoffe von 50 bis 500 reicht.

12. Kolloidale kationische Öl-in-Wasser-Emulsion nach einem der Ansprüche **1** bis **11,** zur Verwendung bei der Behandlung von okularer Hypertension und/oder für die Behandlung von Glaukoma.

13. Abgabevorrichtung, umfassend koloidale Öl-in-Wasser-Emulsion nach einem der Ansprüche **1** bis **12.**

## Revendications

1. Émulsion colloïdale huile-dans-l'eau comprenant :
- une prostaglandine,
- une huile ayant un indice d'iode ≤ 2,
- un ou plusieurs agents tensioactifs comprenant un composé ammonium quaternaire qui est le chlorure de cétalkonium,
- de l'eau,
dans laquelle l'émulsion a un potentiel zêta positif, et un rapport massique prostaglandine/somme des tensioactifs compris entre 0,5 et 5.

2. Émulsion colloïdale huile-dans-l'eau cationique selon la revendication **1, caractérisée en ce que** le rapport massique prostaglandine/somme des tensioactifs est compris entre 0,5 et 4, de préférence entre 0,5 et 3, plus préférentiellement entre 0,5 et 2, encore plus préférentiellement entre 0,6 et 1,5, encore plus préférentiellement entre 0,7 et 1,3, encore plus préférentiellement autour de 1.

3. Émulsion colloïdale huile-dans-l'eau cationique selon les revendications **1** ou **2, caractérisée en ce que** la prostaglandine est choisi parmi une prodrogue de type ester ou amide de prostaglandine active ou un mélange de celles-ci.

4. Émulsion colloïdale huile-dans-l'eau cationique selon l'une quelconque des revendications **1** à **3, caractérisée en ce que** la prostaglandine est choisie parmi latanoprost, isopropyl unoprostone, travoprost, bimatoprost, tafluprost ou un mélange de ceux-ci.

5. Émulsion colloïdale huile-dans-l'eau cationique selon l'une quelconque des revendications **1** à **4, caractérisée en ce que** l'huile est choisie parmi les acides gras, les alcools gras, les esters d'alcools gras comme le myristate d'isopropyle et le palmitate d'isopropyle, les huiles végétales, les huiles animales, les huiles minérales comme le pétrolatum et la paraffine liquide, les huiles semi-synthétiques comme les huiles fractionnées obtenues des huiles végétales, ou un mélanges des ceux-ci.

6. Émulsion colloïdale huile-dans-l'eau cationique la revendication **5, caractérisée en ce que** l'huile fractionnée est un trigycéride à chaîne moyenne (MCT).

7. Émulsion colloïdale huile-dans-l'eau cationique selon l'une quelconque des revendications **1** à **6,** dans laquelle sont en plus inclus des tensioactifs sélectionnés parmi les poloxamers, le tyloxapol, les polysorbates, les esters de sorbitan, les stéarates de polyoxyl ou un mélanges de ceux-ci.

8. Émulsion colloïdale huile-dans-l'eau cationique selon l'une quelconque des revendications **1** à **7,** dans laquelle la quantité de prostaglandine en masse par rapport à la masse totale de l'émulsion est comprise entre 0,001 et 1% m/m, de préférence entre 0,002 et 0,3% m/m, plus préférentiellement entre 0,004 et 0,15% m/m.

9. Émulsion colloïdale huile-dans-l'eau cationique selon l'une quelconque des revendications **1** à **8,** dans laquelle la quantité de chlorure de cétalkonium en masse par rapport à la masse totale de l'émulsion est comprise entre 0,001 et 0,1% m/m, de préférence entre 0,002 et 0,05% m/m, plus préférentiellement entre 0,002 et 0,03% m/m.

10. Émulsion colloïdale huile-dans-l'eau cationique selon l'une quelconque des revendications **1** à **9,** dans laquelle la quantité d'huile en masse par rapport à la masse totale de l'émulsion n'est pas supérieure à 7% m/m, de préférence comprise entre 0,5 et 5% m/m, plus préférentiellement entre 1 et 3% m/m.

11. Émulsion colloïdale huile-dans-l'eau cationique selon l'une quelconque des revendications **1** à **10,** dans laquelle le rapport massique prostaglandine/somme des tensioactifs va de 0,5 à 4 et le rapport massique huile/somme des tensioactifs va de 50 à 500.

12. Émulsion colloïdale huile-dans-l'eau cationique selon l'une quelconque des revendications **1** à **11,** pour son utilisation dans le traitement de l'hypertension oculaire et/ou le traitement du glaucome.

13. Dispositif d'administration comprenant l'émulsion colloïdale huile-dans-l'eau cationique selon l'une quelconque des revendications **1** à **12.**
